# EUROPEAN PATENT APPLICATION

(11) **EP 4 712 099 A1**
(43) Date of publication of application: **18.03.2026**
(21) Application number: 24815463.5
(22) Date of filing: 28.05.2024
(51) Int. Cl.: G16H 50/30, C12Q 1/04, C12Q 1/6888, G16H 10/60

(54) **DEVICE FOR CALCULATING RISK INDICATOR FOR MILD COGNITIVE IMPAIRMENT, METHOD, AND PROGRAM**

(30) Priority: 31.05.2023 JP 2023090352
(71) Applicant: Symbiosis Solutions Inc., Tokyo 101-0064 (JP); Japan Agricultural Frontier Development Organization, Tokyo 101-0064 (JP)
(72) Inventor: MASUYAMA, Hiroaki, Tokyo 101-0064 (JP); HASUKO, Kazumi, Tokyo 101-0064 (JP); OKUMA, Kana, Tokyo 101-0064 (JP); HATAYAMA, Kouta, Tokyo 101-0064 (JP)
(74) Representative: Marks & Clerk LLP
(86) International application number: PCT/JP2024/019474
(87) International publication number: WO 2024/247980

(57) **Abstract**

A technique for calculating a risk of mild cognitive impairment for a subject who submitted a stool sample and a questionnaire , and a system for calculating a risk indicator for mild cognitive impairment is presented. The system includes an extraction unit configured to extract, from a first data related to intestinal microbiota data of a user, a second data related to a factor or intestinal microorganisms that is indicated to relate to the mild cognitive impairment; and a calculation unit configured to input the second data into a model for calculating a probability of the user having the mild cognitive impairment, the model having a third data as a variable, the third data being related to one or more intestinal microorganisms that represents a difference between intestinal microbiota of healthy participants and intestinal microbiota of patients with the mild cognitive impairment, and to calculate the risk of the user for the mild cognitive impairment, wherein the risk for the mild cognitive impairment is a probability of developing or having already developed the mild cognitive impairment.

## Description

### Technical Field

The invention relates to techniques for calculating a disease evaluation index using intestinal microbiota data, and particularly to techniques for calculating a risk of mild cognitive impairment for a subject who submitted a stool sample and a questionnaire.

### Background

Food compositions related to brain health, particularly for maintaining cognitive function, are being developed. Patent Document 1 discloses that L-Ergothioneine is a food composition effective in improving cognitive function in humans.

Additionally, a technique for calculating a disease evaluation index using human intestinal microbiota has been developed. Patent Document 2 discloses a technique for estimating a risk of a disease, such as atopic dermatitis, for a user who submitted a stool sample, using structural equation modeling.

### Related Art

### Patent Documents

Patent document 1: JP 2019-180364 A
Patent document 1: JP 7270143 B

### Summary

### Technical Problem

However, paragraph 0056 of Patent Document 1 only discloses that there is a correlation between a blood concentration of L-Ergothioneine and memory. Therefore, when investigating memory of a subject, it is necessary to collect blood from the subject and to measure the subject's blood concentration of L-Ergothioneine.

One or more embodiments of the instant invention focus on solving such a problem. An object of the embodiments is to provide a technique for calculating a risk of mild cognitive impairment for a subject who submitted a stool sample and a questionnaire.

### Solution to Problem

The first embodiment is a system for calculating a risk indicator for mild cognitive impairment. The system includes an extraction unit configured to extract, from a first data related to intestinal microbiota data of a user, a second data related to a factor or intestinal microorganisms that is indicated to relate to the mild cognitive impairment; and a calculation unit configured to input the second data into a model for calculating a probability of the user having the mild cognitive impairment, the model having a third data as a variable, the third data relating to one or more intestinal microorganisms that represents a difference between intestinal microbiota of healthy participants and intestinal microbiota of patients with the mild cognitive impairment, and to calculate the risk of the user for the mild cognitive impairment, wherein the risk for the mild cognitive impairment represents a probability of developing or having already developed the mild cognitive impairment.

The second embodiment is the system of the first embodiment, wherein the second data is divided into a first group that is more abundant in intestinal microorganisms related to the mild cognitive impairment and a second group that is less abundant in intestinal microorganisms related to the mild cognitive impairment.

The third embodiment is the system of the second embodiment, wherein each of the first group and the second group are subdivided into three groups: males, females, and mixed-sex.

The fourth embodiment is the system of the first embodiment, wherein the second data relates to Mucin Degradation, IgA Protease Production, Promote Serotonin secretion from enterochromaffin cells, Bile acid oxidation, Promotion restoration of intestinal barrier function, HDAC inhibitor production, or Protection of IgA from trypsin degradation.

The fifth embodiment is the system of the first embodiment, wherein the extraction unit also extracts an attribute information of the user; and the calculation unit adds a score to the second data using the user's attribute information and/or information on intestinal microorganisms corresponding to factors related to the mild cognitive impairment, and inputs the scored second data into the model.

The sixth embodiment is the system of the fifth embodiment, wherein the user's attribute information includes background information about the user at the time of the user's birth and information about the user's living background during the user's childhood.

The seventh embodiment is the system of the first embodiment, wherein the model is a model created for each attribute of male and female, or a mixed-sex model.

The eighth embodiment is a method for calculating a risk indicator for mild cognitive impairment, using a computer. The method includes, extracting, from a first data related to intestinal microbiota data of a user, a second data related to a factor or intestinal microorganisms that is indicated to relate to the mild cognitive impairment; and inputting the second data into a model for calculating a probability of the user having the mild cognitive impairment, the model having a third data as a variable, the third data relating to one or more intestinal microorganisms that represents a difference between intestinal microbiota of healthy participants and intestinal microbiota of patients with the mild cognitive impairment, and calculating the risk of the user for the mild cognitive impairment, wherein the risk for the mild cognitive impairment represents a probability of developing or having already developed the mild cognitive impairment.

The ninth embodiment is a program for calculating a risk indicator for mild cognitive impairment, executed by a computer. The program includes, an extraction step of extracting, from a first data related to intestinal microbiota data of a user, a second data related to a factor or intestinal microorganisms that is indicated to relate to the mild cognitive impairment; and a calculation step of inputting the second data into a model for calculating a probability of the user having the mild cognitive impairment, the model having a third data as a variable, the third data relating to one or more intestinal microorganisms that represents a difference between intestinal microbiota of healthy participants and intestinal microbiota of patients with the mild cognitive impairment, and calculating the risk of the user for the mild cognitive impairment, wherein the risk for the mild cognitive impairment represents a probability of developing or having already developed the mild cognitive impairment.

### Advantage

One or more embodiments of the instant invention provide a technique for calculating a risk of a disease that a subject wishes to evaluate for the subject who submitted a stool sample and a questionnaire.

### Brief Description of Drawings

Fig. 1 is a block diagram of a disease evaluation index calculation system in accordance with one or more embodiments.
Fig. 2 is a flowchart of calculating a disease evaluation index in accordance with one or more embodiments.
Fig. 3 is a table showing the questionnaire items for surveying basic information of a large number of subjects and another subject (a user) in accordance with one or more embodiments.
Fig. 4 is a table of conditions for the normal control (NC) group.
Fig. 5 is a table of conditions for the mild cognitive impairment (MCI) group.
Fig. 6 is an explanatory diagram of selecting intestinal microorganism variables in accordance with the first embodiment (the population is females).
Fig. 7 is a table showing the effect size values, application to the association model, and factors related to MCI for each intestinal bacterium in Fig. 6.
Fig. 8 is an explanatory diagram of selecting intestinal microorganism variables in accordance with the second embodiment (the population is males).
Fig. 9 is a table showing the effect size values, application to the association model, and factors related to MCI for each intestinal bacterium in Fig. 8.
Fig. 10 is a hypothetical conceptual diagram of the influence of intestinal bacteria related to MCI.
Fig. 11 is an enlarged view of a part of Fig. 10.
Fig. 12 is an explanatory diagram of the relationship between age and the relative abundance of intestinal bacteria in accordance with the first embodiment (the population is females).
Fig. 13 is an explanatory diagram of score-adding based on the MCI-related factors.
Fig. 14 shows a result of estimating parameters of the association model in accordance with the first embodiment (the population is females).
Fig. 15 is an explanatory diagram of the score estimation model after scoring and the risk calculation model in accordance with the first embodiment (the population is females).
Fig. 16 shows a result of estimating parameters of the association model in accordance with the second embodiment (the population is males).
Fig. 17 is an explanatory diagram of the score estimation model after scoring and the risk calculation model in accordance with the second embodiment (the population is males).
Fig. 18 shows a result of ROC analysis of risk value for MCI in accordance with the first embodiment (the population is females).
Fig. 19 shows a result of ROC analysis of risk value for MCI in accordance with the second embodiment (the population is males).

### Detailed Description of Embodiments

One or more embodiments of the invention are described with reference to the drawings. The same reference numerals are given to common parts in each figure, and duplicate description is omitted.

### (Disease evaluation index calculation system)

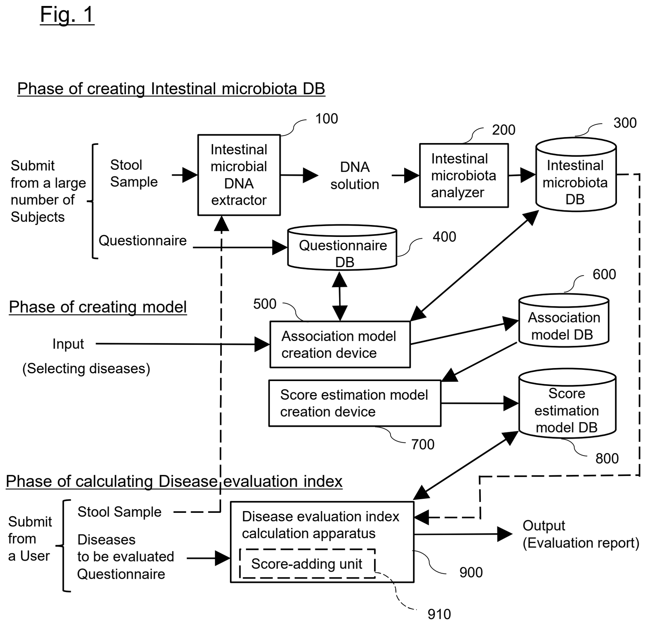

Fig. 1 is a block diagram of a disease evaluation index calculation system in accordance with one or more embodiments. The disease evaluation index calculation system includes a phase of creating an intestinal microbiota database (DB) from stool samples submitted by a large number of subjects, a phase of creating a model relating to one or more diseases (a specific disease), and a phase of calculating an evaluation index of a risk for the specific disease. Here, the large number of subjects are participants who provide samples (their stool samples and data of their questionnaire results) used for the model creation.

In an embodiment, "Mild Cognitive impairment" (hereinafter referred to as MCI) relating to declining cognitive function, is described as an example of the specific disease. The risk for MCI indicates a probability of developing or having already developed MCI. The intestinal microbiota refers to the set (or collection) of intestinal microorganisms that inhabit the human intestines (such as bacteria and archaea), including fungi and yeasts.

### (Phase of creating Intestinal microbiota DB)

In the phase of creating Intestinal microbiota DB, ten thousand or more subjects excrete their stools in toilets and collect their stool samples with stool collection kits. Next, an extractor received the stool samples. The extractor inputs the stool samples into an intestinal microbial DNA extractor 100, and outputs a DNA solution relating to intestinal microbiota. Intestinal microbiota is also called microbiota, human gut microbiota, human intestinal microbiome, or human intestinal flora.

An analyst who received the DNA solution inputs the DNA solution into an intestinal microbiota analyzer 200 and analyzes the intestinal microbiota. The analyst stores the analysis result data of the intestinal microbiota in an intestinal microbiota DB 300. First, the embodiment creates ASV (Amplicon Sequence Variant) sequences that are unique to each subject's intestinal microbiota. Second, the embodiment identifies a taxa name for each subject's intestinal microbiota data using an external DNA sequence database (for example, Ribosomal Database Project database) that is publicly available. The taxa names in the embodiment include taxonomic names of taxonomic ranks (such as family, genus, and species), and taxonomic names used in publicly available external DNA sequence databases.

The intestinal microbiota DB 300 associates the ID (Identification number) information of each subject with the intestinal microbiota of the subject. Although the extractor and the analyst have been described separately, the same business operator may perform the extraction work and the analysis work.

Next, the large number of subjects who submitted their stool samples fill out questionnaires and submit their questionnaire results to a questionnaire collector, using the ID information. The questionnaire collector stores data of the questionnaire results in a questionnaire DB 400.

A questionnaire survey gathers the subjects' detailed information, including the subjects' attribute information, Body Mass Index (BMI), defecation frequency, lifestyle (drinking, smoking, exercise frequency), disease morbidity status (including health status and disease status), sleep status, the Center for Epidemiologic Studies Depression scale (CES-D), presence or absence of Helicobacter pylori treatment, hospitalization or surgery experience, and status for prescription or over-the-counter medications. In the embodiment, the subjects' attribute information refers to basic information such as sex, age, and childhood living area, as the details are described later. Basic information is also called background information.

For females only, the questionnaire survey gathers their menstrual status and whether they were pregnant or breastfeeding. Disease morbidity status was divided into 13 categories (MCI, atopic dermatitis, bone and/or joint disease, bronchial asthma, diabetes, dyslipidemia, gastro-intestinal disease, heart disease, hypertension, kidney disease, liver disease, lower back and/or joint pain, and others) and identified for each patient, including whether or not they were currently being treated. A patient suffering from a disease includes a patient who currently has the disease and/or a patient who has previously had the disease. Prescription or over-the-counter medication use was divided into 16 categories (gastric/duodenal ulcer/reflux esophagitis, hypertension, hyperlipidemia, diabetes, hypnotics, painkillers/antipyretics, allergy, angina treatment, laxative/constipation, osteoporosis, rheumatism, corticosteroids, antibiotics, cold drugs, antithrombotic drugs, and the others) and identified for each patient, including whether or not they were being treated at the time of stool collection.

### (Phase of creating model)

In the model creation phase, first of all, the model creator selects one or more diseases to be evaluated. An association model creation device (or an association modeling device) 500 includes an input unit that inputs MCI as the selected disease, an extraction unit that extracts ID information of subjects related to MCI from the questionnaire DB 400 for each attribute of males and females and extracts intestinal microbiota data of the corresponding ID information from the intestinal microbiota DB 300, and a creation unit that creates an association model using a creation method described later. The association model creation device 500 stores the created association model in an association model DB 600.

Next, the score estimation model creation device 700 extracts the association model from the association model DB 600 and creates a score estimation model using a creation method described later. The created score estimation model is stored in a score estimation model DB 800.

### (Phase of calculating Disease evaluation index)

In the disease evaluation index calculation phase, an evaluator requests a person, who submits the person's stool sample collected by the stool collection kits and wishes to evaluate the risk for MCI (hereinafter referred to as "a user"), to submit the user's stool sample explained in the phase of creating Intestinal microbiota DB. The user's stool sample is processed by the intestinal microbial DNA extractor 100 and the intestinal microbiota analyzer 200, in the same way as the stool samples of the large number of subjects. The intestinal microbiota data of the user is stored in the intestinal microbiota DB 300.

Furthermore, MCI and the user's ID information (including the user's attribute information) are input into the disease evaluation index calculation apparatus 900. Here, the user's attribute information is also input into the disease evaluation index calculation apparatus 900 from the questionnaire result data submitted by the user. The questionnaire result data submitted by the user may also be input into the questionnaire DB 400, in the same way as the questionnaire result data of the large number of subjects. In this case, the disease evaluation index calculation apparatus 900 needs to retrieve the user's questionnaire result data from the questionnaire DB 400. Thus, the user is a person who provides samples (stool sample and questionnaire result data) to request evaluation of the risk for MCI.

The disease evaluation index calculation apparatus 900 includes an input unit that receives input information, an extraction unit that extracts information related to the input information, a score-adding unit that adds one or more scores to the score estimation model, a calculation unit that calculates the evaluation index using the score estimation model after scoring, and an output unit that outputs an evaluation report (or an assessment report). 910 in Fig.1 shows the above-mentioned score-adding unit.

The input unit receives input of MCI as the specific disease and the user's ID information. The extraction unit extracts information (attribute information and intestinal microbiota data of the user) related to the user's ID information from the external intestinal microbiota DB 300, and extracts a score estimation model related to MCI from the score estimation model DB 800.

The score-adding unit 910 adds one or more scores to the score estimation model using the user's attribute information. Then, the calculation unit inputs the user's intestinal microbiota data into the score estimation model after scoring, and calculates the evaluation index of the risk for MCI. The output unit outputs an evaluation report based on the calculated evaluation index. Although business operators who perform each phase have been described separately, the same business operator may perform all the phases.

### (Flowchart of calculating a disease evaluation index)

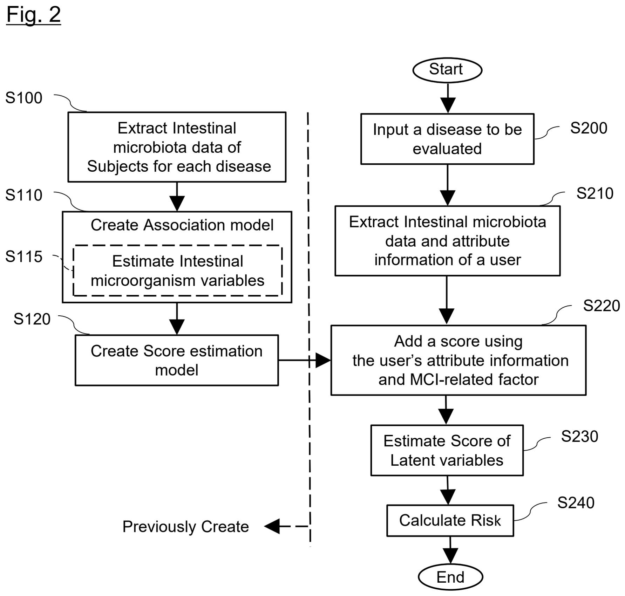

Fig. 2 is a flowchart of calculating a disease evaluation index in accordance with the embodiment. These processes are divided into two process groups. The first process group (S100 through S120) creates score estimation models for each disease in advance. The second process group (S200 through S240) calculates the evaluation index of the risk for MCI, which is the disease the user wishes to evaluate.

The first process group includes a step of extracting intestinal microbiota data of a plurality of subjects for each disease (S100), a step of creating an association model for each disease (S110), and a step of creating a score estimation model for each disease (S120). The second process group includes a step of inputting MCI as the disease the user wishes to evaluate (S200), a step of extracting the user's intestinal microbiota data (S210), a step of adding one or more scores to the score estimation model using the user's attribute information and/or information on intestinal bacteria corresponding to factors related to MCI (S220), a step of estimating score of latent variables using the score estimation model after scoring (S230), and a step of calculating the evaluation index of the risk for MCI (S240).

### (Description of S100)

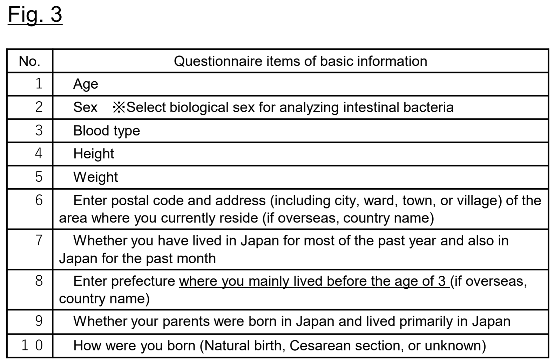

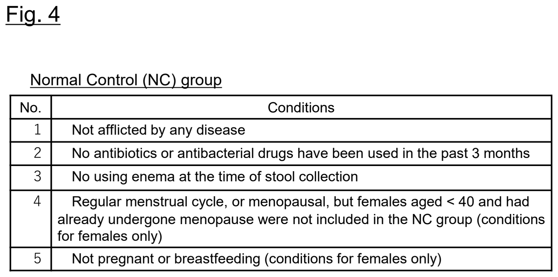

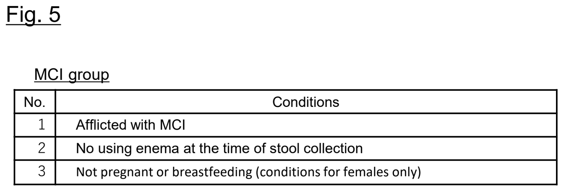

In the embodiment, "MCI" is described as an example of the disease. S100 extracts intestinal microbiota data of a group of healthy participants (or disease-free controls) and a group of patients with MCI (or participants suffering from MCI) from the intestinal microbiota DB 300. The people belonging to these groups correspond to the above-mentioned subjects. Fig. 3 is a table showing the questionnaire items for surveying basic information of the subjects and the user. Fig. 4 and Fig. 5 show conditions for extracting each group. These conditions include conditions for females only. It should be noted that the questionnaire items in Fig. 3 are to be surveyed not only for the subjects but also for the user.

Fig. 3 is a table showing the questionnaire items for surveying basic information of the subjects and the user in the embodiment. The embodiment is a technique for calculating the disease evaluation index for MCI using the intestinal microbiota data of the user. Intestinal microbiota of the subjects and the user may be influenced by their age and biological sex (hereinafter simply referred to as sex), and their living background, such as the area in which they spent their childhood. The questionnaire items No. 8 and No. 9 relate to their living background. Additionally, the item No. 10 surveys about the method of delivery as a factor for inheriting their mother's intestinal microbiota. Therefore, the information that may be related to the intestinal microbiota data of the subjects and the user is referred to as their basic information.

In the embodiment, the attribute information of the subjects and the user is surveyed using the questionnaire items shown in Fig. 3. To survey the influence of their living background on their intestinal microbiota, for example, they are asked about the prefecture where they mainly lived before the age of 3.

The precondition of the embodiment is that all of the subjects and the user are Japanese. Therefore, their countries of birth are not surveyed. However, surveying the country of birth may be added to the questionnaire items. Hereinafter, the basic information of the subjects and the user extracted from the questionnaire result data is referred to as their "attribute information". The attribute information includes the background at the time of birth of the subjects and the user, and the living background during their childhood.

Fig. 4 is a table of conditions for the normal control (NC) group. The normal control group (Normal Control, hereinafter referred to as NC) is a group of healthy participants. The NC group consists of participants who satisfied all conditions shown in Fig. 4. Fig. 5 is a table of conditions for the MCI group. The group of patients with MCI (hereinafter referred to as MCI group) is a group of participants who satisfied all conditions shown in Fig. 5. In the embodiment, each group is further divided into males and females. The MCI group consists of 26 male participants aged between 40 and 80 years old and 33 female participants aged between 40 and 80 years old.

### (Description of S110)

S110 is a step of creating an association model for MCI. The embodiment creates association models for MCI separately for males and females. In the embodiment, the male population includes intestinal microbiota data from 11 patients in their 70s with MCI and 17 healthy participants in their 70s, and the female population includes intestinal microbiota data from 18 patients in their 70s with MCI and 23 healthy participants in their 70s. The reason for limiting the MCI group to patients in their 70s is that this age group has the highest number among patients between the ages of 40 and 80. Even if human intestinal microbiota includes the same intestinal microorganisms, the same intestinal microorganisms may behave differently in human bodies of males and females.

First, the intestinal microbiota data extracted in S100 undergoes a centered log-ratio (CLR) transformation. The count values of the intestinal microbiota data are transformed into CLR values. Processing this transformation makes the following statistical process easier.

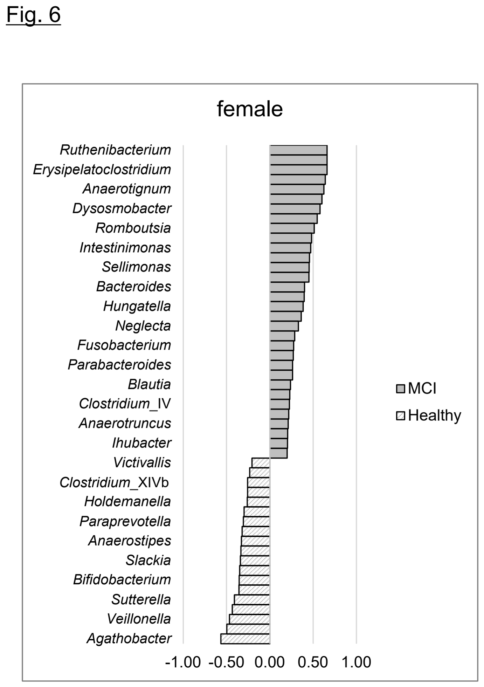

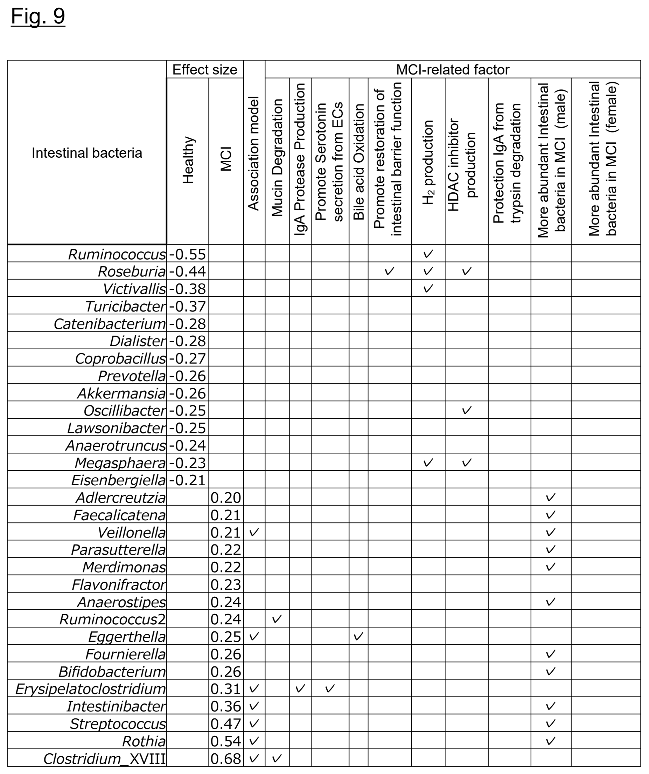

Next, the embodiment performs a step of estimating intestinal microorganism variables (S115). The intestinal microorganism variables refer to the intestinal microorganisms that are indicated to be related to MCI. S115 is conducted to search for one or more intestinal that represent the differences between the normal control (NC) group and the MCI group. In the embodiment, intestinal bacteria are selected based on the effect size between the NC and the MCI groups for each sex population. Intestinal bacteria with effect size values greater than 0.2 are defined as more abundant in the MCI group compared to the NC group, while intestinal bacteria with effect size values less than -0.2 are defined as less abundant in the MCI group. Since the populations are divided by sex, the intestinal bacteria that are more abundant in the MCI group and the other intestinal bacteria that are less abundant in the MCI group differ between males and females (see the following fig. 6 and fig. 9). Fig. 6 and the subsequent figures cite intestinal bacteria as an example of intestinal microorganisms.

Fig. 6 is an explanatory diagram of selecting intestinal microorganism variables in accordance with the first embodiment (the population is females). The horizontal axis shows the effect size, while the vertical axis shows the names of intestinal bacteria (at genus level). For the convenience of creating the bar graphs, intestinal bacteria with positive effect sizes are represented as "MCI", while intestinal bacteria with negative effect sizes are represented as "Healthy". Thus, intestinal bacteria with positive effect sizes are more abundant in the intestinal microbiota of the MCI group, while intestinal bacteria with negative effect sizes are less abundant in the intestinal microbiota of the MCI group. Therefore, the processing of S110 can estimate multiple intestinal bacteria that are estimated to contribute to the differences in the intestinal microbiota of the NC and the MCI groups. It should be noted that, although there are 51 types of the actual intestinal bacteria, Fig. 6 shows only 26 types in order to make the bar graphs easier to read.

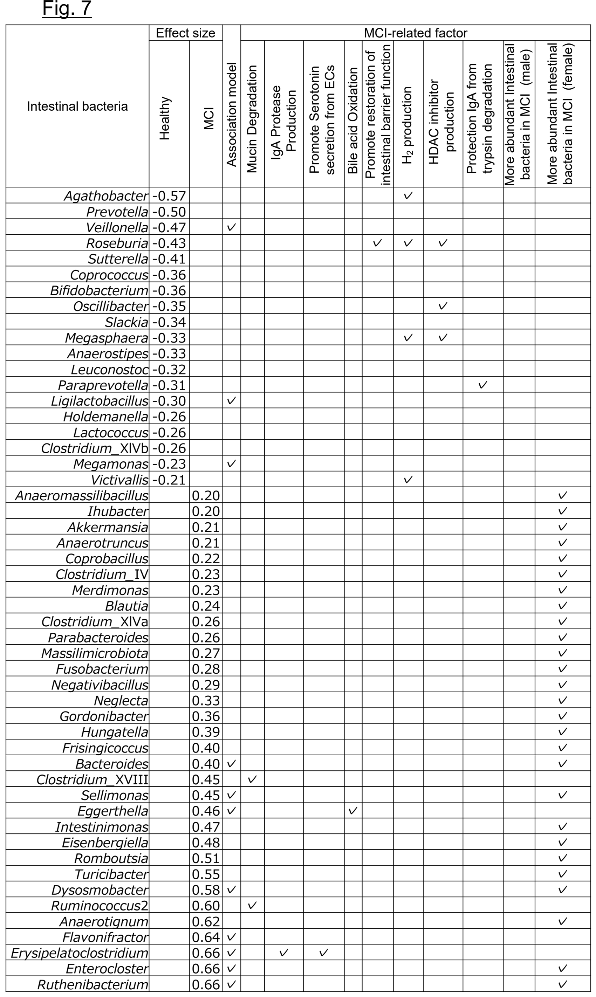

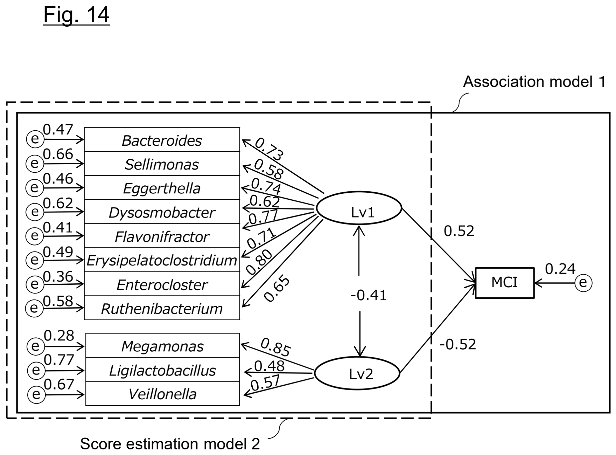

Figure 7 is a table showing the effect size values, application to the association model, and factors related to MCI for the above-mentioned 51 types of intestinal bacteria. The check marks show whether each of the intestinal bacteria is applicable to the association model, and MCI-related factors to which each of the intestinal bacteria corresponds. MCI-related factors refer to factors or intestinal bacteria (or intestinal microorganisms) that are indicated to be related to MCI. Also, the intestinal bacteria marked with a check in the column of the "Association model" are described later in Fig. 14.

In Fig. 7, ten factors related to MCI are listed. Specifically, the MCI-related factors are 1) Mucin Degradation, 2) IgA Protease Production, 3) Promote Serotonin secretion from enterochromaffin cells (ECs), 4) Bile acid oxidation, 5) Promotion restoration of intestinal barrier function, 6) Hydrogen (H₂) production, 7) HDAC inhibitor production, 8) Protection of IgA from trypsin degradation, 9) Intestinal bacteria that are more abundant in MCI (in the case of male), 10) Intestinal bacteria that are more abundant in MCI (in the case of female). In the embodiment, the MCI-related factors are classified into ten categories. However, the MCI-related factors are not limited to the ten categories and may be one or more categories. Furthermore, the MCI-related factors may be other MCI-related factors.

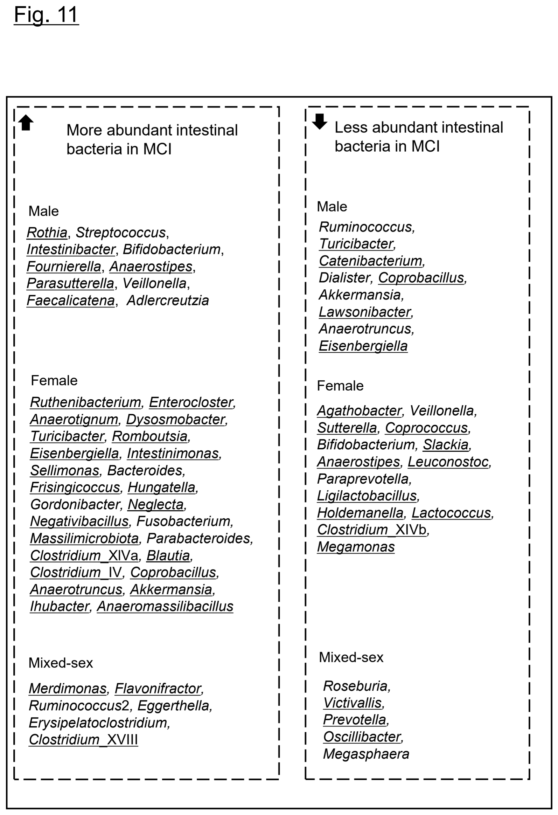

The intestinal bacteria listed in the column of effect size "Healthy" have numerical values, and some of the intestinal bacteria have no check mark on any MCI-related factors. They correspond to other MCI-related factors. For example, Prevotella corresponds to "Less abundant intestinal bacteria in MCI (mixed-sex)" (see the following fig. 11). Moreover, Flavonifractor, which has numerical values listed in the column of effect size "MCI," corresponds to "More abundant intestinal bacteria in MCI (mixed-sex)" (see the following fig. 11). In the case of Fig. 7, since the population is female, there is no check mark on the item "More abundant intestinal bacteria in MCI (male)". Furthermore, although the names of intestinal bacteria are originally written in italics, it should be noted that the names are written in a normal font, due to the character code available for use in the specification.

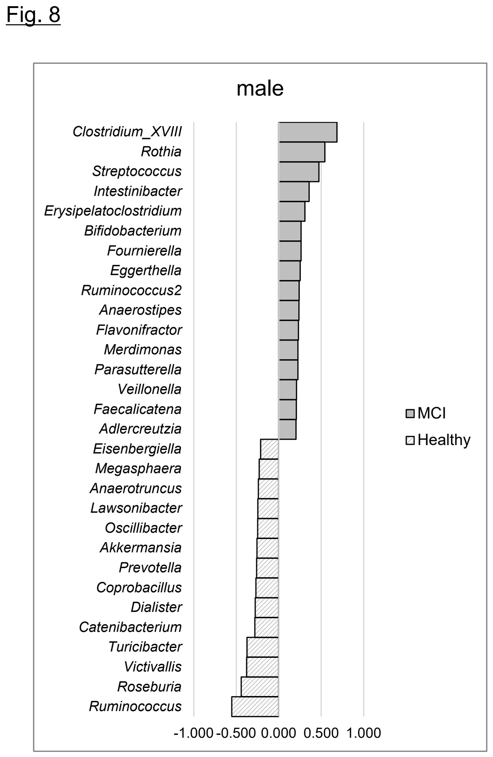

Fig. 8 and Fig. 9 correspond to Fig. 6 and Fig. 7 in the case that the population is males. That is, Fig. 8 is an explanatory diagram of selecting intestinal microorganism variables in accordance with the second embodiment (the population is males). Fig. 9 is a table showing the effect size values, application to the association model described later, and factors related to MCI for each intestinal bacteria shown in Fig. 8. In the case of Fig. 9, since the population is male, there is no check mark on the item "More abundant intestinal bacteria in MCI (female)". Furthermore, the reason that there is no check mark on the item "Protection IgA from trypsin degradation" is because the corresponding intestinal bacteria does not appear in Fig. 9.

The embodiment confirms whether or not all of a plurality of the intestinal bacteria estimated in S110 correspond to the MCI-related factors shown in Fig. 7. In the embodiment, by performing the confirmation, it becomes possible to carry out the process of adding one or more scores described later.

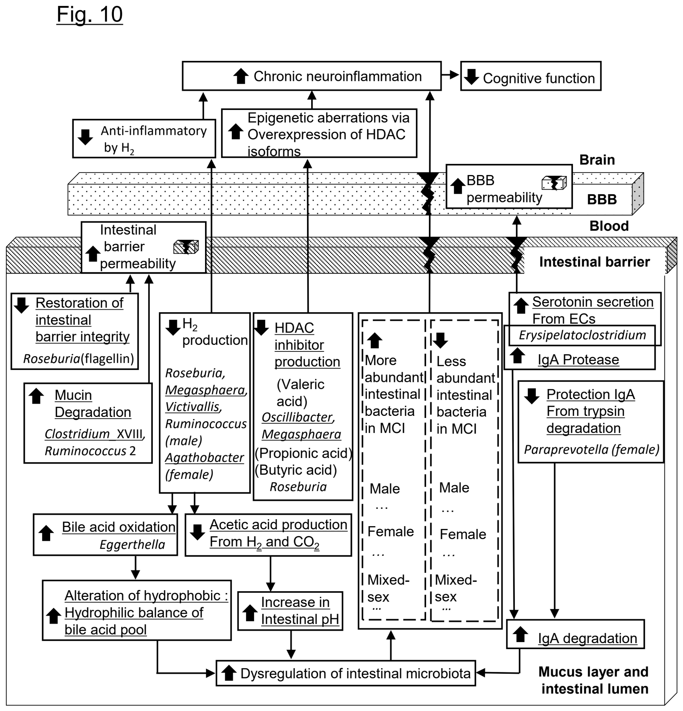

First, the MCI-related factors shown in Fig. 7 are described using Fig. 10. Fig. 10 is a hypothetical conceptual diagram of the influence of intestinal microbiota related to MCI. The bold upward arrows in the figure indicate an increase. The bold downward arrows indicate a decrease. For example, the "IgA degradation" is marked with an upward bold arrow. In this case, it means that "IgA degradation is promoted."

In the embodiment, a comparative study is conducted in advance to lead to the hypotheses shown in Fig. 10. Specifically, the embodiment investigated the association between MCI and intestinal microbiota by comparing the MCI group aged 70s (including 11 males and 18 females) and the NC group (including 17 males and 23 females), taking sex into account. For a plurality of intestinal bacteria related to MCI that are revealed by the comparative study, an additional literature review is conducted on known characteristics, such as hydrogen production. Some of intestinal bacteria are not described in the literature as being related to MCI. However, based on some known characteristics and the comparative study, it is hypothesized that the composition of intestinal microbiota in the MCI group leads to dysregulation of intestinal microbiota, increased intestinal barrier permeability, increased Blood-Brain Barrier permeability (BBB permeability), and increased chronic neuroinflammation, with the long-term persistence of these abnormalities ultimately leading to cognitive decline.

Additionally, the comparative study is conducted separately by sex, and it is set that an intestinal bacteria is related to the MCI group if the absolute value of the effect size of the intestinal bacteria is 0.2 or more. In the intestinal bacteria of Fig. 10, there are cases where "(male) or (female)" is indicated next to the name of the intestinal bacteria. This means "More abundant intestinal bacteria in MCI (male) or More abundant intestinal bacteria in MCI (female)."

In the embodiment, in addition to the analysis of mixed-sex groups, a re-analysis of the groups by sex is performed to understand the association between MCI and intestinal microbiota. As a result, although it is a hypothesis, it becomes possible to explain a part of the mechanisms underlying MCI associated with intestinal bacteria as shown in Fig. 10. By leading to such a hypothesis, the embodiment is able to set MCI-related factors and construct a model to calculate the probability of a user developing or having already developed MCI. Furthermore, the embodiment creates an association model for MCI and a score estimation model, and then a risk calculation model, as will be described later. This risk calculation model corresponds to a model that calculates the probability of a user developing or having already developed MCI.

Fig. 10 is a hypothetical conceptual diagram of a part of the mechanisms underlying MCI associated with intestinal microbiota. The following hypotheses is about the influence of the intestinal microbiota related to MCI by category.
(1) A decrease in the abundance of H₂ producing bacteria Roseburia, Megasphaera, Victivallis, Ruminococcus (male), and Agathobacter (female), and an increase in the abundance of Eggerthella, which oxidizes bile acids in a H₂ concentration-dependent manner, lead to dysregulation of the intestinal microbiota.
(2) A decrease in the abundance of H₂ producing bacteria Roseburia, Megasphaera, Victivallis, Ruminococcus (male), and Agathobacter (female) leads to dysregulation of the intestinal microbiota by indirectly causing an increase in intestinal pH due to decreased acetic acid production.
(3) An increase in the abundance of IgA protease-producing Erysipelatoclostridium and a decrease in the abundance of Paraprevotella (female), which protects IgA via the degradation of intestinal trypsin, leads to dysregulation of the intestinal microbiota and increased inflammation of intestinal epithelial cells.
(4) An increase in the abundance of Clostridium_XVIII and Ruminococcus 2, which are associated with mucin degradation, and a decrease in the abundance of Roseburia (flagellin), associated with the protection of intestinal barrier function, leads to increased intestinal barrier permeability.
(5) An increase in the abundance of Erysipelatoclostridium, which promotes serotonin secretion in the intestine, leads to increased BBB permeability.
(6) The taxa of intestinal bacteria that are more abundant due to dysregulation of the intestinal microbiota differ between male and female, but they contribute to inflammation.
(7) A decrease in the abundance of HDAC (histone deacetylase) inhibitor-producing bacteria (such as Oscillibacter and Megasphaera, which produce valeric acid, Roseburia, which produces propionic acid and butyric acid) leads to epigenetic dysregulation due to excess HDAC activity, leading to increased inflammation.
(8) A decrease in the abundance of H₂ producing bacteria Roseburia, Megasphaera, Victivallis, Ruminococcus, and Agathobacter leads to decreased reactive oxygen species removal by H₂ and increased chronic inflammation.
(9) The composition of the intestinal microbiota in MCI-affected individuals leads to dysregulation of the intestinal microbiota, increased intestinal barrier and BBB permeability, and increased chronic neuroinflammation, which, when sustained over time, ultimately leads to cognitive decline.

"More abundant intestinal bacterial (group) in MCI" and "Less abundant intestinal bacterial (group) in MCI" shown in Fig. 10 that are revealed by the above comparative study, are further subdivided into three subgroups: male, female, and mixed-sex. Due to space limitations, these classifications are shown as an enlarged view in Fig. 11. The underlined "item names" and "intestinal bacterial names" in Fig. 10 and Fig. 11 are not found in the above literature review. For example, it is known that hydrogen is involved in MCI. However, although the intestinal bacteria Megasphaera, Victivallis, and Agathobacter (female) are known for their H₂ production ability, this study is the first to reveal that these intestinal bacteria are associated with (or contributes to) MCI. Therefore, these intestinal bacteria are emphasized with underlines.

In the embodiment, it is possible to set MCI-related factors based on the hypotheses of the mechanisms underlying MCI associated with intestinal bacteria. More specifically, the items listed with the names of intestinal bacteria shown in Fig. 10 and Fig. 11 can be set as MCI-related factors.

### (Example 1 of Score-adding process; score-adding using attribute information)

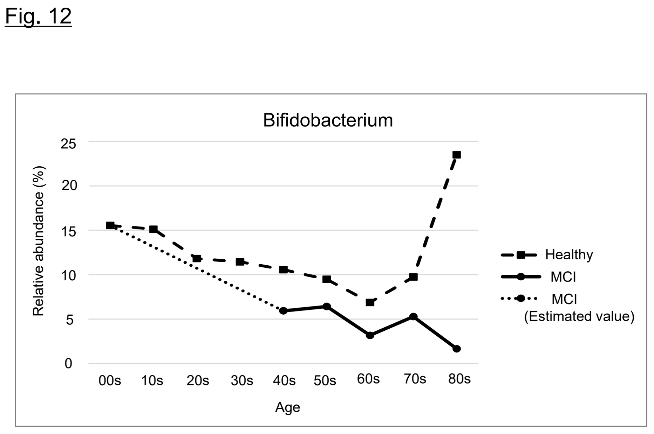

Fig. 12 is an explanatory diagram of the relationship between age and the abundance rate of all female subjects for Bifidobacterium, that is one of the intestinal bacteria with a negative effect size of the all female subjects shown in Fig. 6. In Fig. 12, "Healthy" represents an average value for each age in the NC group, while "MCI" represents another average value for each age in the MCI group. When the subjects are younger, there are no patients with MCI. Therefore, for ages in which data on patients with MCI cannot be measured, estimated values are used as the abundance rate for those ages (shown as "MCI (estimated value)" in Fig. 12). A relative abundance refers to the proportion of a certain intestinal bacterium in the intestinal microbiota.

The onset of MCI is associated with the accumulation of damage to the brain. Furthermore, the intestinal microbiota changes with age. As will be described later, the embodiment may perform score-adding process using attribute information such as the age of the user.

Fig. 12 shows that the judgment of whether the relative abundance of Bifidobacterium is appropriate or not is dependent on the age of the female user. For example, it is assumed that the user is 60 years old. The average value for 60 years old in Fig. 12 shows that "Healthy" is 6.9% and "MCI" is 3.2%. Therefore, if the relative abundance of Bifidobacterium in the intestinal microbiota of the user is 6.9% or more, score-adding process is not applied. On the other hand, if the relative abundance of the user is 3.2% or less, score-adding process is applied to Bifidobacterium.

### (Example 2 of Score-adding process; score-adding to the intestinal bacteria that corresponds to MCI-related factors)

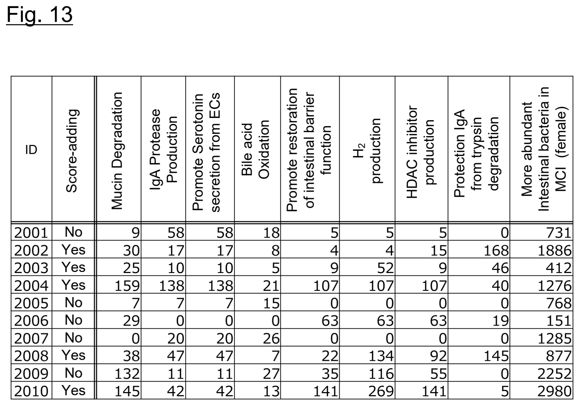

The embodiment may apply score-adding to the intestinal bacteria that corresponds to MCI-related factors among the plurality of the intestinal bacteria estimated in S110, if the user possesses all of the MCI-related factors among the plurality of the intestinal bacteria estimated in S110. Fig. 13 is an explanatory diagram of score-adding based on the possessed bacteria in accordance with the embodiment. The figure shows cases in which the subjects in the MCI group are females. Therefore, the figure excludes "More abundant intestinal bacteria in MCI (male)" from the MCI-related factors. Among the subjects in the MCI group shown in the figure, who have been added scores (which are different from zero, and are the abundance rate or number of sequences) to all of the MCI-related factors (that is, when the intestinal microbiota of the subjects possesses all of the MCI-related factors), the score-adding process is applied to the corresponding possessed bacteria. In the embodiment, when all of the MCI-related factors are possessed, it is considered to accelerate association with MCI, and score-adding is applied; however, when a part of MCI-related factors are possessed, score-adding may be applied.

Returning to the process of S110, the step of creating an association model using the intestinal bacteria estimated in S115 is described below. The embodiment creates an association model using the CLR-transformed intestinal microbiota data and MCI morbidity status data of the NC and the MCI groups.

Fig. 14 shows a result of estimating parameters of the association model in accordance with the first embodiment. The population is females. The embodiment uses Structural Equation Model (SEM) as an association model. SEM represents relationships between observed variables and latent variables, and relationships between the latent variables. Therefore, the association model is a statistical analysis model constructed by observed variables that have a certain degree of correlation with a specific disease and latent variables that are related to the observed variables.

The association model 1 is an area surrounded by the solid line. Rectangles show the observed variables. "e" is a residual variance. Ellipses show the latent variables. Numerical values of arrows that are from the latent variables to the observed variables or the other latent variables show standardized parameters of the association model. For example, the observed variable "MCI" is an observed variable for the disease "MCI" and is defined as a binary categorical variable representing MCI morbidity status. When a participant is afflicted with MCI, the observed variable "MCI" is "1 (one)." When the other participant is not afflicted with MCI (or healthy), the observed variable "MCI" is "0 (zero)."

The embodiment assumes that there are two latent variables that explained the binary categorical variables, in case of creating the association model. The latent variable 1 (Lv 1) has a positive effect on MCI. The latent variable 2 (Lv 2) has a negative effect on MCI.

First, all of the intestinal bacteria shown in Fig. 6 are assigned as observed variables related to latent variables Lv1 or Lv2 (also referred to as intestinal microorganism variables). This is the first hypothesized association model. The embodiment uses the first hypothesized association model as a starting point to modify the model. The step of modifying the model conducts to delete observed variables until no negative components appeared in the variance-covariance matrix calculated in the process of the structural equation modeling.

Subsequently, observed variables are deleted so that p-values (significance probability) of each parameter is less than 0.05. The final association model is selected from the constructed models, on the condition that the values of Goodness-of-Fit Index (GFI) and Adjusted GFI (AGFI) are close to "1", the value of Root Mean Square Error of Approximation (RMSEA) is close to "0", and the absolute value for the path coefficient from the latent variable to the disease morbidity variable representing disease morbidity is maximum.

The embodiment merges the data for the NC and the MCI groups as the intestinal microbiota data and the MCI morbidity status data. The embodiment inputs the merged data to the created association model, and conducts to calculate each parameter of the association model. The embodiment investigates how the intestinal bacteria that are set as the observed variables explains the variables representing MCI morbidity status, using the assumed latent variables (Lv1, Lv2), when focusing on the NC and the MCI groups.

The standardized path coefficient from latent variable Lv1, which is hypothesized to have a positive effect on MCI, to the MCI morbidity variable is "0.52" (p<0.01). The standardized path coefficient from latent variable Lv2, which is hypothesized to have a negative effect on MCI, to the MCI morbidity variable is "-0.52" (p<0.01). The standardized factor loading values from the latent variable Lv1 to each of the observed variables (intestinal microorganism variables) are, for example, "0.65" for Ruthenibacterium. The standardized factor loading values from the latent variable Lv2 to each of the observed variable (intestinal microorganism variables) are, for example, "0.85" for Megamonas. The standardized factor loading values from the latent variables to each of the observed variables (intestinal microorganism variables) are all significant at p<0.05. The numerical value of the doubleheaded arrow between the latent variables that is "-0.41" represents the correlation coefficient.

The residual variance of the MCI morbidity variable is "0.24," and the association model 1 shows the result that the assumed latent variables (Lv1, Lv2) explain approximately 76% of the variance in the MCI morbidity variable.

In the embodiment, S115 is an example of a method for selecting observed variables for an association model, and selecting intestinal microorganism variables using effect sizes as indexes. S115 may not be required.

In the following, the relationship between the observed variables related to the latent variables (Lv1, Lv2) in Fig. 14 and the intestinal bacteria marked in the "Association model" column in Fig. 7 is explained. The observed variables related to the latent variable Lv1 in Fig. 14 have numerical values in the effect size column "MCI" in Fig. 7, and are marked with a check in the "Association model" column in Fig. 7. Similarly, the observed variables related to the latent variable Lv2 have numerical values in the effect size column "Healthy", and are marked with a check in the "Association model" column. Thus, the embodiment proved that the intestinal bacteria that are marked with a check in the "Association model" column in Fig. 7 are applied in the association model of the embodiment.

There are various ways to select observed variables for an association model. For example, some statistical tests (such as Wilcoxon rank sum test) may be performed between the NC and the MCI groups, and intestinal bacteria that show a significant difference in abundance between the two groups may be used as an observed variable in the association model. Alternatively, intestinal bacteria that have already been reported to be a potential biomarker for the disease being evaluated may be used as an observed variable in the association model.

### (Description of S120)

S120 is a step of creating a model for estimating scores of latent variables, when an MCI morbidity status is unknown. The embodiment uses the merged data for the NC and the MCI groups (that is, the intestinal microbiota data and the MCI morbidity status data), in case of creating an association model. However, for a user who wishes to evaluate a risk for MCI, the user's disease morbidity status is unknown. Therefore, the embodiment needs a model for estimating scores of latent variables, when an MCI morbidity status is unknown.

When a female user's MCI morbidity status is unknown, the embodiment extracts a measurement equation model (a part surrounded by the dotted line in Fig. 14) from the association model 1 and creates a score estimation model 2, in order to estimate scores of Lv 1 and Lv 2. Therefore, the embodiment sets each parameter value of the measurement equation model to each parameter value of the score estimation model 2. Here, the measurement equation model shows the influence of the latent variables on the observed variables of the intestinal bacteria (intestinal microorganism variables). The score estimation model 2 of the embodiment is a linear multiple regression model that is created in which the observed variables of the intestinal bacteria (intestinal microorganism variables) are set to explanatory variables and scores of latent variables (Lv1 and Lv2) are set to objective variables.

The embodiment estimates the score of the user's Lv1 and Lv2, after processing score-adding to the score estimation model 2 using the user's attribute information and/or information on intestinal bacteria corresponding to MCI-related factors. In the following, the estimated values of the latent variables Lv1 and Lv2 are represented as Lvlest and Lv2est, respectively.

### (Description of S200)

S200 is a step of inputting a disease that a user wishes to evaluate. The embodiment creates score estimation models for MCI separately for sex. The user also inputs the user's sex. In the embodiment, the user is female.

### (Description of S210)

S210 is a step of extracting the user's intestinal microbiota data and attribute information. The user submits the user's stool sample, and the user's intestinal microbiota data is stored in the intestinal microbiota DB 300. S210 extracts the user's intestinal microbiota data from the intestinal microbiota DB 300 using the user's ID information. S210 also extracts the user's attribute information (age, childhood living area, etc.) from the questionnaire result data submitted by the user.

### (Description of S220)

S220 is a step of adding one or more scores to the score estimation model using the user's attribute information and/or information on intestinal bacteria corresponding to factors related to MCI. The user's attribute information is input into the disease evaluation index calculation apparatus 900.

The score-adding unit 910 calculates the composite variable x (x1 = attribute, x2 = living area, ...) from the user's attribute information. Next, the composite variable x is input into the predetermined scoring function y = f(x), and the value y output by the scoring function is added to the intestinal microorganism variables related to Lv1, which are marked with a check in "MCI-related factors" column in Fig. 7 (for example, Erysipelatoclostridium in Fig. 14).

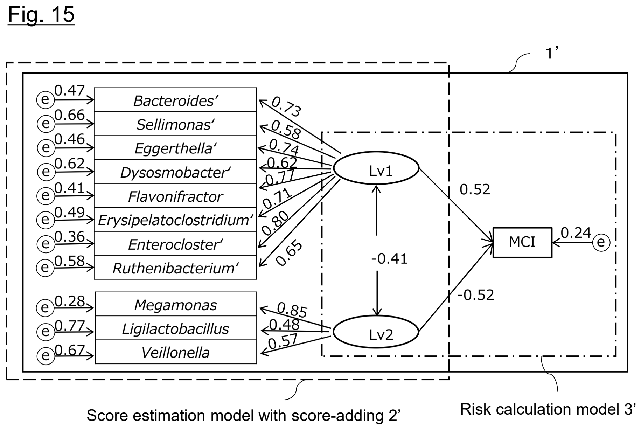

Fig. 15 is an explanatory diagram of the score estimation model with score-adding and the risk calculation model in accordance with the first embodiment. Each intestinal microorganism variable with an apostrophe in Fig. 15 (for example, Erysipelatoclostridium') is the intestinal microorganism variable (observed variable) after score-adding. For example, "Erysipelatoclostridium'" means "Erysipelatoclostridium' = Erysipelatoclostridium + y."

Among the variables related to Lv1, "Flavonifractor" is not added a score and is not added an apostrophe, because "Flavonifractor" is not marked with a check in the column of "MCI-related factors" in Fig. 7. The embodiment applies score-adding process to a part of the variables related to Lv1, but depending on the setting of MCI-related factors, Score-adding process may be applied to all of the variables related to Lv1. MCI-related factors may be set to apply score-adding process to all of the latent variables (Lv1 and Lv2).

A part surrounded by the dotted line in Fig. 15 is "Score estimation model with score-adding 2'". Another part surrounded by the dash-dotted line in Fig. 15 is "Risk calculation model 3'" described later. It should be noted that the entire part 1' in Fig. 15 has changed variable values for each intestinal microorganism variable in the association model of Fig. 14. For example, the variable value of the intestinal microorganism variable "Erysipelatoclostridium" has been changed to "Erysipelatoclostridium + y."

### (Description of S230)

S230 is a step of estimating scores of latent variables using the score estimation model with score-adding 2'. The score-adding is related to MCI. Specifically, from the first data related to the user's intestinal microbiota, the embodiment extracts the second data related to factors or intestinal microorganisms indicated to be related to MCI. The embodiment inputs the second data to each observed variable of the "score estimation model 2'" (for example, Erysipelatoclostridium') and estimates the user's score of the latent variables (Lv1, Lv2) in the "score estimation model 2'." The estimated values of the latent variables Lv1 and Lv2 are set to Lvlest and Lv2est. That is, by setting the intestinal microorganisms with score-adding into variables, S230 inputs the second data to the model for calculating the probability of a user with a disease (the score estimation model 2'), and estimates the user's scores of the latent variables Lv1, Lv2 in the score estimation model 2'. Here, the intestinal microorganisms with score-adding may be referred to as the second data after adding a score. In the embodiment, the first data corresponds to the data related to the user's intestinal microbiota. The second data corresponds to the intestinal microorganisms of the user, which are marked with a check in the "Association model" column in Fig. 7. The third data corresponds to the intestinal microorganisms of the subjects, which are marked with a check in the "Association model" column. That is, the third data corresponds to one or more intestinal microorganisms that show the differences between the intestinal microbiota of healthy participants and the intestinal microbiota of patients with mild cognitive impairment.

### (Description of S240)

S240 is a step of calculating an evaluation index of the risk for MCI (or calculating the evaluation index numerically representing the risk for MCI). Specifically, the embodiment calculates the user's risk for MCI, using the path coefficients from the latent variables Lv1 and Lv2 in the risk calculation model 3' in Fig. 15 to the observed variable MCI and the estimated values of the latent variables (Lvlest and Lv2est). That is, S240 calculates the user's risk for MCI in the risk calculation model 3' based on the probability calculated by the score estimation model 2' (the user's scores of the latent variables Lv1 and Lv2 in the score estimation model 2').

The method for constructing the MCI morbidity probability estimation model uses a logistic regression model that has latent variables Lv1 and/or Lv2 as explanatory variables, and binary categorical variables representing a user's MCI morbidity status as objective variables. This logistic regression model corresponds to the risk calculation model 3' (the dash-dotted line in Fig. 15).

The disease evaluation index calculation apparatus 900 outputs an evaluation report to the user based on the calculated risk for MCI.

The above explains the flowchart of calculating a disease evaluation index in Fig. 2. In the case where the population is female, the association model 1 related to MCI shows a correlation of "-0.41" between the latent variables Lv1 and Lv2 in Fig. 14. On the other hand, when the population is male, there is a correlation between Lv1 and Lv2 and it is assumed that there is one latent variable. The following describes the case where there is one latent variable as a variation of the embodiment.

### (Variation of the embodiment; when there is one latent variable)

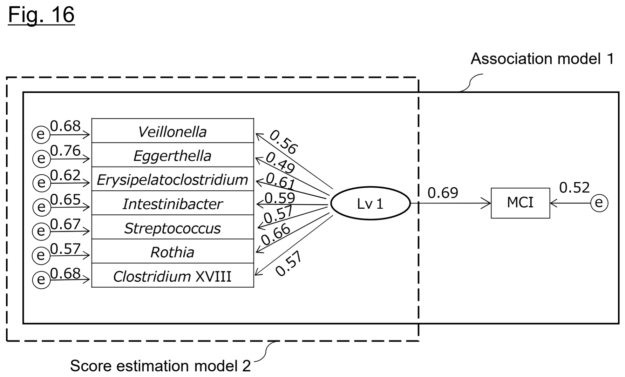

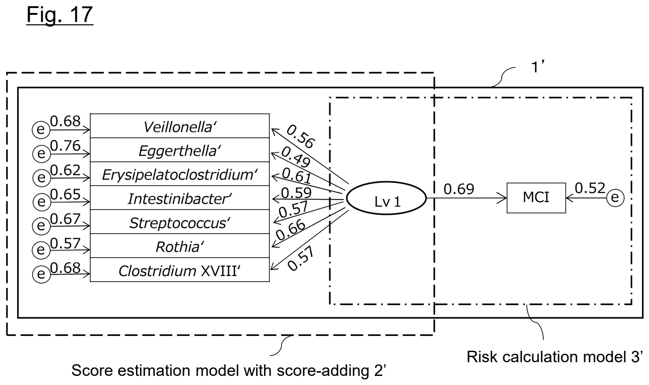

Fig. 16 shows a result of estimating parameters of the association model 1 in accordance with the second embodiment. The population is males. Fig. 17 is an explanatory diagram of the "score estimation model with score-adding 2'" and the "risk calculation model 3'" in accordance with the second embodiment. The second embodiment shows that there is one latent variable. All of the variables related to Lv1 in Fig. 17 have a check mark on one of the items in the "MCI-related factor" column, all of the variables are scored, and an apostrophe is added to each of the variables.

Even in the case of one latent variable, the processes S110 (create association model), S120 (create score estimation model), S220 (Add a score using the user's attribute), S230 (estimate scores of latent variables), and S240 (calculate risk) in Fig. 2 can be performed as described above.

### (An example of calculating risk)

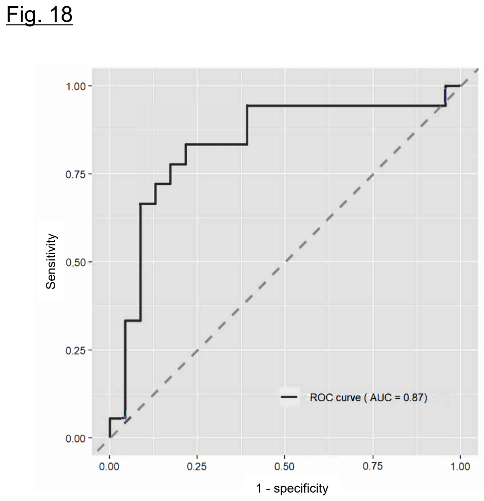

The calculation of risk for MCI is explained separately for the first embodiment (the population is females) and the second embodiment (the population is males). Fig. 18 shows a result of ROC analysis of the risk value for MCI in accordance with the first embodiment (the population is females). The following describes the risk calculation and the accuracy of the calculated risk in accordance with the first embodiment (the population is females).

The merged groups of the NC, and the MCI groups in the female subjects are randomly divided in a stratified manner into 80% as a training group and 20% as a verification group. The embodiment learns an association model using scores of latent variables (Lv1 or Lv2) and MCI morbidity status data in the training group, and constructs an MCI morbidity probability estimation model that estimates MCI morbidity probability from the scores of the latent variables. The embodiment applies the estimated values of the latent variable scores (Lvlest or Lv2est) in the verification group to the constructed MCI morbidity probability estimation model, and investigates the accuracy of the MCI morbidity probability estimation model using ROC (Receiver Operating Characteristic) analysis. The MCI morbidity probability is a risk for getting MCI, and the result of the ROC analysis is used as a disease evaluation index.

The method for constructing the MCI morbidity probability estimation model uses a logistic regression model that has latent variables Lv1 and/or Lv2 as explanatory variables, and binary categorical variables representing a user's MCI morbidity status as objective variables. The logistic regression model corresponds to the "risk calculation model 3'" in Fig. 15.

Fig. 18 shows ROC curves for the logistic regression model. The vertical axis shows "Sensitivity." As the probability of identifying a patient who actually suffers from MCI as one who has MCI becomes higher, the value on the vertical axis approaches "1". The horizontal axis shows "1 - Specificity". As the probability of identifying a patient who does not suffer from MCI as one who does not have MCI becomes higher, the value of "Specificity" approaches "1". The horizontal axis represents "1 - (minus) Specificity". As the value on the horizontal axis approaches "0", the probability of a patient who does not suffer from MCI being identified as one who does not have MCI becomes higher. The solid line represents the results of the model using Lv1 and Lv2 as explanatory variables (described in Fig. 14 and Fig. 15, the population is females). AUC represents the value of Area Under the Curve, which is "0.87" in the case of the embodiment (the population is females).

In Fig. 18, the diagonal line (the dotted line) represents a probability of "0.5". In the case of the model using Lv1 and Lv2 as explanatory variables (the solid line), the area enclosed by the solid line and the dotted lines is wide. That can be visually (or intuitively) determined that the accuracy of the risk estimation method is high. AUC is an index of the risk estimation method. When AUC value is close to 1, that can be objectively determined to be a good risk estimation method. The embodiment is judged to be highly accurate as an indirect risk estimation method, excluding direct risk estimation methods such as blood tests.

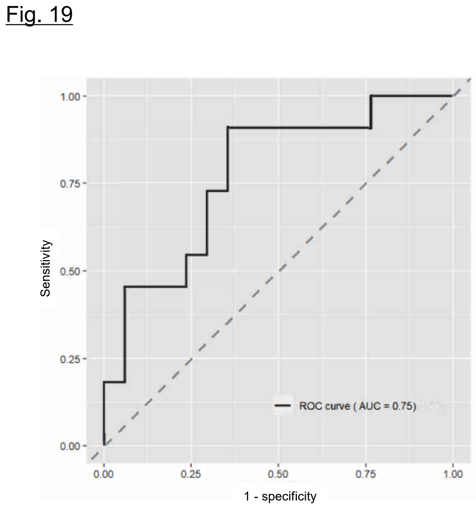

Fig. 19 shows a result of ROC analysis of risk value for MCI in accordance with the second embodiment (the population is males). The AUC is "0.75." Therefore, similar to the first embodiment (the population is females), the second embodiment can be judged to be highly accurate as an indirect risk estimation method, excluding direct risk estimation methods such as blood tests.

### (Effects)

According to the embodiment, the association model for each disease (for example, the association model 1 in Fig. 14) is created using the intestinal microbiota data and disease morbidity status data of the large number of subjects, and the score estimation model (for example, the score estimation model 2 in Fig. 14) is created in advance from a measurement equation portion of the association model. For a user wishing to evaluate the risk for MCI as a specific disease, the embodiment processes individually based on the user's stool sample and questionnaire result data submitted by the user, similar to the large number of subjects.

The embodiment extracts the user's intestinal microbiota data, using the stool sample submitted by the user. The embodiment also extracts the user's attribute information (including the background at the time of the user's birth, and the living background in the user's childhood), using the questionnaire result data submitted by the user. The embodiment applies score-adding to score estimation models for MCI (for example, the score estimation model 2 in Fig. 14), using the user's attribute information. By inputting the user's intestinal microbiota data into the observed variables of the score estimation model with score-adding (for example, "Score estimation model with score-adding 2'" in Fig. 15), the embodiment estimates the score of the latent variable for the user. The embodiment calculates the user's risk for MCI, using the estimated user's score and the risk calculation model (for example, "Risk calculation model 3'" in Fig. 15).

Therefore, the user receives an evaluation report regarding the user's risk of developing or having already developed MCI in a non-invasive and easy manner. The business operator who provides the evaluation report may design, study, and propose probiotics to the user.

For example, by including prescriptions for functional foods corresponding to the user's intestinal microbiota data in the evaluation report, in addition to the user's risk for MCI, the evaluator provides cognitive function improvement solutions to the user.

### (Cognitive function improvement solution)

The evaluation report for the user may also introduce functional food that is expected to improve the cognitive function of the user, in addition to the user's risk for MCI. The embodiment introduces food for improving cognitive function corresponding to the user's intestinal microbiota data.

The embodiment identifies, among these cognitive function improvement foods, a functional food (cognitive function improvement food) that contributes to the improvement to MCI by increasing the intestinal microbiota according to the user's intestinal microbiota data. The embodiment provides (or prescribes) a report on the intake method of the identified functional food for the user. The user regularly consumes the identified functional food according to the intake method. Then, after a few months, by using the cognitive function index calculation system of the embodiment again as a follow-up observation, the user can utilize the cognitive function improvement solution. The cognitive function improvement solution provides the user with an evaluation report before and after the intake of the functional food.

For example, the observed variable "MCI" in the association model 1 of Fig. 14 is defined as the observed variable related to the disease "MCI", which is a binary categorical variable representing the MCI morbidity status, but is not limited to this. The embodiment may set the observed variable "MCI" to a continuous value of 0 to 100, and may perform an intervention trial to confirm the increase or decrease of MCI when the user consumes a functional food that can affect the intestinal microorganism variables that are factors (risk factors or preventive factors) of MCI.

The above-mentioned embodiments (including variations or modified examples) of the invention have been described. Furthermore, two or more of the embodiments may be combined. Alternatively, one of the embodiments may be partially implemented. Furthermore, two or more of the embodiments may be partially combined.

Furthermore, embodiments of the invention are not limited to the description of the above embodiments. Various modifications are also included in the embodiments of the invention as long as a person skilled in the art can easily conceive without departing from the description of the embodiments.

For example, a number of latent variables is assumed to be 1 (one) or 2 (two). the number may be 3 (three) or more. The embodiment extracts ID information of subjects related to the disease from the questionnaire DB 400 for each attribute of male and female, and creates the association model, but is not limited to this. The embodiment may create the model for mixed-sex without extracting for each attribute of male and female.

S230 uses the score estimation model with score-adding, in case of estimating scores of the latent variables, but is not limited to this. The embodiment may use the score estimation model before score-adding, without adding a score.

Moreover, the intestinal microorganisms that are indicated to be related to MCI may include the following: Acidaminococus, Adlercreutzia, Agathobacter, Akkermansia, Anaeromassilibacillus, Anaerostipes, Anaerotignum, Anaerotruncus, Bacteroides, Bifidobacterium, Blautia, Catenibacterium, Clostridium IV, Clostridium sensu stricto, Clostridium XIVa, Clostridium X1Vb, Clostridium XVIII, Coprobacillius, Coprobacter, Coprococcus, Dialister, Dysosmobacter, Eggerthella, Eisenbergiella, Enterocloster, Enterococcus, Erysipelatoclostridium, Faecalicatena, Flavonifractor, Fournierella, Frisingicoccus, Fusobacterium, Gordonibacter, Holdemanella, Holdemania, Hungatella, Ihubacter, Intestinibacter, Intestinimonas, Lactococcus, Lawsonibacter, Leuconostoc, Ligilactobacillus, Longicatena, Massilimicrobiota, Megamonas, Megasphaera, Merdimonas, Negativibacillus, Neglecta, Oscillibacter, Parabacterioides, Paraprevotella, Parasutterella, Prevotella, Romboutsia, Roseburia, Rothia, Ruminococcus, Ruminococcus 2, Ruthenibacterium, Sellimonas, Slackia, Streptococcus, Sutterella, Turicibacter, Unclassified (DNA sequences without assigned taxonomic names), Veillonella, and Victivallis.

The embodiment uses an association model, but is not limited to this, and may be performed by machine learning. When an association model is used, the disease evaluation index calculation apparatus in accordance with the embodiment includes an input unit configured to input one or more diseases that a user wants to evaluate; an extraction unit configured to extract a first intestinal microbiota data of healthy people and a second intestinal microbiota data of people with the disease, using a first database that stores intestinal microbiota data that results from analyzing stool samples of a plurality of subjects, a second database that stores result data of a questionnaire on the one or more diseases for the plurality of subjects, and predetermined extraction conditions; a first creation unit configured to input the first intestinal microbiota data, the second intestinal microbiota data, and the result data of the questionnaire, and to create an association model including a first observed variable related to a plurality of intestinal microorganisms that express differences between the first intestinal microbiota data and the second intestinal microbiota data, a second observed variable representing a morbidity status of the disease, one or more latent variables relating to the first observed variable and the second observed variable, and parameters being from the one or more latent variables to the first and second observed variables and/or being between the latent variables; a second creation unit configured to create a score estimation model including the first observed variable as an explanatory variable, and a score of the latent variable as an objective variable; a scoring unit configured to input the user's attribute information and to add a score to a part of the explanatory variable of the score estimation model using the user's attribute information; an estimation unit configured to input the user's intestinal microbiota data to the explanatory variable of the score estimation model with score-adding and to estimate the score of the latent variable; and a calculation unit configured to calculate the user's risk for the disease using parameters from the one or more latent variables to the second observed variables and/or the latent variables, and the estimated score. Among these structural elements, the first creation unit, the second creation unit, the scoring unit, the estimation unit, and the calculation unit may be performed by machine learning.

### Reference Signs List

- 100: Intestinal microbial DNA extractor
- 200: Intestinal microbiota analyzer
- 300: Intestinal microbiota DB
- 400: Questionnaire DB
- 500: Association model creation device
- 600: Association model DB
- 700: Score estimation model creation device
- 800: Score estimation model DB
- 900: Disease evaluation index calculation apparatus
- 910: Score-adding unit

## Claims

1. A system for calculating a risk indicator for mild cognitive impairment, comprising:
an extraction unit configured to extract, from a first data related to intestinal microbiota data of a user, a second data related to a factor or intestinal microorganisms that is indicated to relate to the mild cognitive impairment; and
a calculation unit configured to input the second data into a model for calculating a probability of the user having the mild cognitive impairment, the model having a third data as a variable, the third data being related to one or more intestinal microorganisms that represents a difference between intestinal microbiota of healthy participants and intestinal microbiota of patients with the mild cognitive impairment, and to calculate the risk of the user for the mild cognitive impairment,
wherein
the risk for the mild cognitive impairment represents a probability of developing or having already developed the mild cognitive impairment.

2. The system of claim 1, wherein the second data is divided into a first group that is more abundant in intestinal microorganisms related to the mild cognitive impairment and a second group that is less abundant in intestinal microorganisms related to the mild cognitive impairment.

3. The system of claim 2, wherein each of the first group and the second group is subdivided into three groups: males, females, and mixed-sex.

4. The system of claim 1, wherein the second data relates to Mucin Degradation, IgA Protease Production, Promote Serotonin secretion from enterochromaffin cells, Bile acid oxidation, Promotion restoration of intestinal barrier function, HDAC inhibitor production, or Protection of IgA from trypsin degradation.

5. The system of claim 1, wherein the extraction unit also extracts an attribute information of the user; and
the calculation unit adds a score to the second data using the user's attribute information and/or information on intestinal microorganisms corresponding to factors related to the mild cognitive impairment, and inputs the scored second data into the model.

6. The system of claim 5, wherein the user's attribute information includes background information about the user at the time of birth and living background information about the user during the user's childhood.

7. The system of claim 1, wherein the model is a model created for each attribute of male and female, or a mixed-sex model.

8. A method for calculating a risk indicator for mild cognitive impairment, using a computer, comprising:
extracting, from a first data related to intestinal microbiota data of a user, a second data related to a factor or intestinal microorganisms that is indicated to relate to the mild cognitive impairment; and
inputting the second data into a model for calculating a probability of the user having the mild cognitive impairment, the model having a third data as a variable, the third data being related to one or more intestinal microorganisms that represents a difference between intestinal microbiota of healthy participants and intestinal microbiota of patients with the mild cognitive impairment, and calculating the risk of the user for the mild cognitive impairment,
wherein the risk for the mild cognitive impairment represents a probability of developing or having already developed the mild cognitive impairment.

9. A program for calculating a risk indicator for mild cognitive impairment, executed by a computer, the program comprising:
an extraction step of extracting, from a first data related to intestinal microbiota data of a user, a second data related to a factor or intestinal microorganisms that is indicated to relate to the mild cognitive impairment; and
a calculation step of inputting the second data into a model for calculating a probability of the user having the mild cognitive impairment, the model having a third data as a variable, the third data being related to one or more intestinal microorganisms that represents a difference between intestinal microbiota of healthy participants and intestinal microbiota of patients with the mild cognitive impairment, and calculating the risk of the user for the mild cognitive impairment,
wherein the risk for the mild cognitive impairment represents a probability of developing or having already developed the mild cognitive impairment.
